(19) Europäisches Patentamt
European Patent Office
Office européen des brevets

(11) Veröffentlichungsnummer: **0 562 510 A1**

# (12) EUROPÄISCHE PATENTANMELDUNG

(21) Anmeldenummer: **93104654.4**

(22) Anmeldetag: **22.03.93**

(51) Int. Cl.[5]: **C07D 239/60**, C07D 251/30, C07D 405/12, A01N 43/54

(30) Priorität: **27.03.92 DE 4209931**

(43) Veröffentlichungstag der Anmeldung: **29.09.93 Patentblatt 93/39**

(84) Benannte Vertragsstaaten: **AT BE CH DE DK ES FR GB IT LI NL SE**

(71) Anmelder: **HOECHST AKTIENGESELLSCHAFT**

**D-65926 Frankfurt(DE)**

(72) Erfinder: **Zurmühlen, Frank, Dr.**
**Inselbergstrasse 13**
**W-6230 Frankfurt am Main 80(DE)**
Erfinder: **Bauer, Klaus, Dr.**
**Doorner Strasse 53d**
**W-6450 Hanau(DE)**
Erfinder: **Bieringer, Hermann, Dr.**
**Eichenweg 26**
**W-6239 Eppstein/Ts.(DE)**

(54) **Optisch akfive Pyrimidinyl- oder Triazinyl-oxy-(oder -thio)-carbonsäurederivate, Verfahren zur ihrer Herstellung und Verwendung als Herbizide oder Pflanzenwachstumsregulatoren.**

(57) Gegenstand der Erfindung sind optisch aktive Pyrimidinyl- oder Triazinyl-oxy-(oder - thio)-carbonsäurederivate der allgemeinen Formel (I),

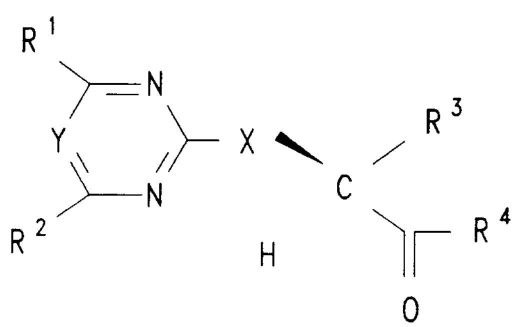

(I)

worin
X, Y, $R^1$, $R^2$, $R^3$ und $R^4$ wie in Anspruch 1 definiert sind, bedeuten, in einer Enantiomerenreinheit der L-Form von größer als Null.

Die erfindungsgemäßen Verbindungen der Formel (I) weisen eine ausgezeichnete herbizide Wirksamkeit gegen ein breites Spektrum von Schadpflanzen auf.

EP 0 562 510 A1

Es ist bekannt, daß viele Naturstoffe mit biologischer Wirksamkeit infolge des Vorhandenseins eines oder mehrerer asymmetrischer Kohlenstoffatome optisch aktiv sind, d. h. die Ebene des polarisierten Lichtes nach rechts (+) oder links (-) drehen. Sehr oft weisen diese Verbindungen eine höhere biologische Wirksamkeit auf als entsprechende synthetisch gewonnene, optisch inaktive Verbindungen. Ähnliches gilt für manche synthetisch hergestellte Wirkstoffe wie Pharmazeutika oder Pflanzenschutzmittel mit asymmetrischen C-Atomen, die gewöhnlich bei der Synthese als optisch inaktive Racemate, d. h. Gemische aus gleichen Anteilen der rechts- und linksdrehenden Enantiomeren, anfallen. Auch hier wird bei der Isomerentrennung nicht selten gefunden, daß die Wirksamkeit eines der beiden Enantiomeren höher ist als die des Racemats. Ob die links- oder rechtsdrehende Form die jeweils aktive ist und ob überhaupt ein Zusammenhang zwischen Wirkung und optischer Aktivität besteht, läßt sich jedoch in der Regel nicht vorhersagen.

Aus der Klasse der Pyrimidinyloxycarbonsäurederivate wurden in letzter Zeit Verbindungen bekannt, die wegen ihren herbiziden und pflanzenwachstumsregulierenden Eigenschaften von Interesse sind; siehe z. B. EP-A-0347811, EP-A-0409369, EP-A-0409368, EP-A-0400741. Diese besitzen in Nachbarstellung ("α-Stellung" oder 2-Position) zur Carbonsäurederivat-Gruppe ein asymmetrisches C-Atom, so daß zwei enantiomere, optisch aktive Formen existieren, die nach der Nomenklatur unter Anwendung der Fischer-Projektion gewöhnlich als D- und L-Form und nach der Cahn-Ingold-Prelog-Nomenklatur (s. Angew. Chem. 78 (1966) 413 ff.) als Enantiomere mit (R)- oder (S)-Konfiguration, genauer (2R)- bzw. (2S)-Konfiguration bezeichnet werden.

Es wurde nun gefunden, daß die L-Enantiomeren einiger dieser Verbindungen sich durch eine erheblich höhere herbizide Wirkung im Vergleich zu den Racematen auszeichnen.

Gegenstand der Erfindung sind somit optisch aktive Pyrimidinyl- oder Triazinyl-oxy-(oder -thio)-carbonsaurederivate der allgemeinen Formel (I),

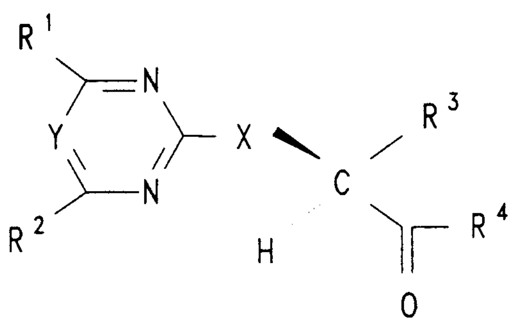

(I)

worin

X O oder S,

Y N oder CH,

$R^1$, $R^2$ unabhängig voneinander Wasserstoff, Alkyl, Alkoxy, Alkylthio, Halogen, Haloalkoxy, Amino, Alkylamino oder Dialkylamino,

$R^3$ ein aliphatischer oder araliphatischer Rest der Formel

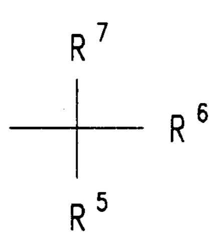

oder

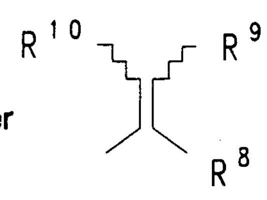

$R^5$, $R^6$ unabhängig voneinander Wasserstoff oder Alkyl oder $R^5$ und $R^6$ gemeinsam mit dem verbundenen C-Atom einen 3- bis 6-gliedrigen Ring, der ein Sauerstoffatom enthalten kann und durch ein oder mehrere Alkylgruppen substituiert sein kann,

$R^7$ Wasserstoff, Halogen, Alkyl, das unsubstituiert oder durch ein oder mehrere Reste aus der Gruppe Halogen, Hydroxy, Alkoxy und Alkylthio substituiert ist, oder Cycloalkyl, Hydroxy, Cyano, Aryl oder Arylalkyl, wobei die letztgenannten beiden Reste im Aryl-Teil unsubstituiert oder substituiert sind,

$R^8$ Alkyl, Phenyl oder substituiertes Phenyl oder $R^8$ gemeinsam mit $R^{10}$ und der verbundenen Ethenylen-Gruppe einen Cyclopenten- oder Cyclohexenring, der unsubstituiert oder durch ein oder mehrere Alkylgruppen substituiert ist,

$R^9$ Wasserstoff oder Alkyl,

$R^{10}$ unabhängig voneinander Wasserstoff oder Alkyl oder $R^{10}$ gemeinsam mit $R^8$ und der verbundenen Ethenylen-Gruppe einen Cyclopenten- oder Cyclohexenring, der unsubstituiert oder durch ein oder mehrere Alkylgruppen substituiert ist,

$R^{11}$ Wasserstoff, Halogen, Nitro, Alkyl, Haloalkyl, Alkoxy, Alkylsulfonyl, Alkylsulfinyl oder Alkylthio,

$R^4$ Hydroxy, $-O^{-}Kat^{+}$, einen aliphatischen gesättigten oder ein- oder mehrfach ungesättigten Kohlenwasserstoffoxyrest, vorzugsweise mit ein bis 24 C-Atomen, insbesondere ein Rest aus der Gruppe Alkoxy, Alkenyloxy und Alkinyloxy,
wobei der Rest unsubstituiert oder durch ein oder mehrere Reste aus der Gruppe Halogen, Alkoxy, Alkylthio, Alkoxycarbonyl, Alkylcarbonyl, Phenyl, Phenoxy, Phenylthio, wobei die drei letztgenannten Reste unsubstituiert oder substituiert sind, Aminocarbonyl,
Alkylaminocarbonyl und Dialkylaminocarbonyl substituiert ist, oder Phenoxy, substituiertes Phenoxy, Alkylidenaminoxy, Cycloalkyloxy oder Halogen oder
$R^4$ den Rest eines 5- oder 6-Ringlactons, vorzugsweise eines Lactonrestes, der die Bindungsstelle in $\alpha$-Position zur CO-Gruppe hat, oder
$R^4$ einen Rest der Formel

$-D^1-CR^{12}R^{13}-D^2-L$ oder

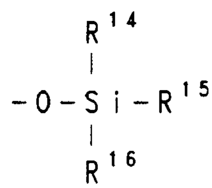

$Kat^+$ das Äquivalent eines ein-, zwei- oder mehrwertigen Kations,

$R^{12}$, $R^{13}$ unabhängig voneinander Wasserstoff, Alkyl, Alkenyl, Alkinyl, Aryl, substituiertes Aryl, Benzyl, Alkoxy, Alkenyl, Alkinyloxy, Aryloxy oder substituiertes Aryloxy,

$R^{14}$, $R^{15}$, $R^{16}$ unabhängig voneinander Alkyl, Alkenyl oder Alkinyl, Phenyl oder substituiertes Phenyl,

$D^1$ O, S oder $NR^{17}$,

$R^{17}$ Wasserstoff oder Alkyl,

$D^2$ eine direkte Bindung oder eine gesättigte oder ungesättigte Kohlenwasserstoffgruppe,

L eine Säurederivat-Rest der Formel

$- CO - O - R^{18}$ oder $- CO - S - R^{18}$ und

$R^{18}$ Wasserstoff, Alkyl, Alkenyl oder Alkinyl, wobei die letztgenannten 3 Reste unsubstituiert oder durch ein oder mehrere Reste aus der Gruppe Halogen substituiert sind, Cycloalkyl, Cycloalkenyl, Benzyl, Phenyl oder substituiertes Phenyl

bedeuten, in einer Enantiomerenreinheit der L-Form von größer als Null.

Für die Klassifizierung der Verbindungen der Formel (I) als L-Form oder L-Enantiomer ist hier analog der üblichen Einordnung von D- und L-Glyzerinaldehyd oder D- und L-aminosäuren die Orientierung des Pyrimidinyl- oder Triazinyl-oxy-(oder -thio)-Rest als Bezugsligand in der horizontalen Ebene der Fischerprojektionsformel maßgebend, wobei die Säurederivatgruppe oberhalb und die Gruppe der Formel $R^3$ unterhalb der genannten horizontalen Ebene und jeweils in der vertikalen Ebene angeordnet sind.

Die L-Form entspricht bei den Verbindungen der Formel (I) der (2S)-Konfiguration gemaß der Cahn-Ingold-Prelog-Nomenklatur.

Unter dem genannten Begriff "Enantiomerenreinheit" in Prozent [% ee], der auch als "Enantiomerenüberschuß" (englisch: "enantiomeric excess") bezeichnet wird der folgende Quotient bezeichnet (siehe z. B. B. Testa, "Grundlagen der Stereochemie", Verlag Chemie GmbH, Weinheim 1983, Seiten 144 - 145):

% ee = [(L - D)/(L + D)] x 100

In der vorstehenden mathematischen Formel bedeuten "L" und "D" die Konzentrationen der L- bzw. der D-Form in dem jeweiligen Gemisch der Enantiomeren. Bevorzugt liegen die Verbindungen der Formel (I) in einer L-Enantiomerenreinheit von 40 bis 100 Prozent, besser von 60 bis 100 Prozent, insbesondere von 80 bis 100 Prozent vor. Dies entspricht einem Anteil von vorzugsweise 70 bis 100 Prozent L-Form, besser 80 bis 100 Prozent L-Form, insbesondere 90 bis 100 Prozent L-Form, bezogen auf das Gemisch der D- und L-Enantiomeren.

In der Formel (I) und im folgenden können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod. Haloalkyl bedeutet Alkyl, das durch ein oder mehrere gleichartige oder verschiedenartige Atome aus der Gruppe Halogen substituiert ist; Haloalkyl ist beispielsweise $CF_3$, $CHF_2$, $CH_2CF_3$, $CCl_3$, $CClF_2$, $CFCl_2$, $CF_2CF_3$. Entsprechendes gilt für Haloalkenyl, Haloalkoxy und andere durch Halogen substituierte Reste. Aryl bedeutet beispielsweise Phenyl, Naphthyl, Tetrahydronaphthyl, Indenyl, Indanyl, Pentalenyl, Fluorenyl und ähnliches, vorzugsweise Phenyl; Aryloxy bedeutet vorzugsweise die den genannten Arylresten entsprechenden Oxy-Reste, insbesondere Phenoxy. Heteroaryl bzw. Heteroaryl in Heteroaryloxy bedeutet beispielsweise Pyridyl, Pyrimidyl, Pyridazyl, Thienyl, Furyl, Pyrrolyl, Pyrazolyl und Imidazolyl, vorzugsweise Pyridyl und Thienyl, aber auch bicyclische oder polycyclische aromatische oder araliphatische Verbindungen. Gegebenenfalls substituiertes Aryl bzw. Aryloxy, Heteroaryl, Heteroaryloxy, Phenyl, Phenoxy, Benzyl, Benzyloxy bzw. gegebenenfalls substituierte bicyclische Reste mit aromatischen Anteilen bedeuten vorzugsweise jeweils den entsprechenden unsubstituierten Rest oder bedeuten einen davon abgeleiteten substituierten Rest, wobei die Substituenten beispielsweise ein oder mehrere, vorzugsweise 1, 2 oder 3 Reste aus der Gruppe Halogen, Alkyl, Haloalkyl, Alkoxy, Haloalkoxy, Nitro, Cyano, Alkoxycarbonyl, Alkylcarbonyl, Alkanoyl, Carbamoyl, Mono- und Dialkylaminocarbonyl, Mono- und Dialkylamino, Alkylsulfinyl und Alkylsulfonyl bedeuten und bei Resten mit C-Atomen solche mit 1 bis 4 C-Atomen, insbesondere 1 oder 2, bevorzugt sind. Bevorzugt sind dabei in der Regel Substituenten aus der Gruppe Halogen, z. B. Fluor und Chlor, $C_1$-$C_4$-Alkyl, vorzugsweise Methyl oder Ethyl, $C_1$-$C_4$-Haloalkyl, vorzugsweise Trifluormethyl, $C_1$-$C_4$-Alkoxy, vorzugsweise Methoxy oder Ethoxy, $C_1$-$C_4$-Haloalkoxy, Nitro und Cyano. Besonders bevorzugt sind dabei die Substituenten Methyl, Methoxy und Chlor.

Beispiele für siliciumhaltige Rest $R^4$ sind Trialkylsilyalkoxy, Aryldialkylsilylalkoxy, Diarylalkylsilyalkoxy, Trialkylsililylalkenyloxy, Aryldialkylsilylalkenyloxy, Diarylalkylsilylalkenyloxy, Trialkylsililylalkinyloxy, Aryldialkylsilyalkinyloxy, Diarylalkylsilylakindyloxy, Trialkylsilyloxy, Aryldialkylsilyloxy, Diarylalkylsilyloxy.

Von besonderem Interesse sind erfindungsgemäße Verbindungen der Formel (I), worin

$R^1$, $R^2$ unabhängig voneinander $C_1$-$C_4$-Alkyl, $C_1$-$C_4$-Alkoxy, Halogen, $C_1$-$C_4$-Haloalkoxy, $C_1$-$C_4$-Alkylamino oder Di-($C_1$-$C_4$-alkyl)-amino, vorzugsweise Methyl, Methoxy, Chlor oder Methylamino, insbesondere einer von beiden Resten Methoxy und der andere Rest Methyl, Methoxy, Chlor oder Methylamino

bedeuten.

Von besonderem Interesse sind auch erfindungsgemäße Verbindungen der Formel (I), worin

$R^5$, $R^6$ unabhängig voneinander Wasserstoff oder $C_1$-$C_4$-Alkyl oder $R^5$ und $R^6$ gemeinsam mit dem verbundenen C-Atom einen 3- bis 6-gliedrigen Ring, der ein Sauerstoffatom enthalten kann und durch ein oder mehrere $C_1$-$C_4$-Alkyl substituiert sein kann,

$R^7$ Wasserstoff, Halogen, $C_1$-$C_4$-Alkyl, das unsubstituiert oder durch ein oder mehrere Reste aus der Gruppe Halogen, Hydroxy, $C_1$-$C_4$-Alkoxy und $C_1$-$C_4$-Alkylthio substituiert ist, oder $C_3$-$C_8$-Cycloalkyl, Hydroxy, Cyano, Thienyl, Naphthyl, Dihydronaphthyl, wobei die letztgenannten drei Reste unsubstituiert oder substituiert sind, oder einen Rest der Formel

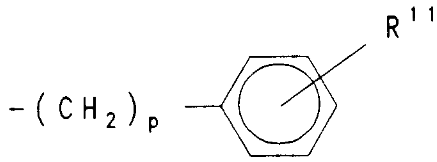

p 0, 1, 2 oder 3,

$R^8$ $C_1$-$C_4$-Alkyl, Phenyl oder substituiertes Phenyl oder $R^8$ gemeinsam mit $R^{10}$ und der verbundenen Ethenylen-Gruppe einen Cyclopenten- oder Cyclohexenring, der unsubstituiert oder durch ein oder mehrere $C_1$-$C_4$-Alkyl substituiert ist,

$R^9$ Wasserstoff oder $C_1$-$C_4$-Alkyl,

$R^{10}$ unabhängig voneinander Wasserstoff oder $C_1$-$C_4$-Alkyl oder $R^{10}$ gemeinsam mit $R^8$ und der verbundenen Ethenylen-Gruppe einen Cyclopenten- oder Cyclohexenring, der unsubstituiert oder durch ein oder mehrere $C_1$-$C_4$-Alkyl substituiert ist,

$R^{11}$ Wasserstoff, Halogen, Nitro, $C_1$-$C_{12}$-Alkyl, $C_1$-$C_4$-Haloalkyl, $C_1$-$C_4$-Alkoxy, $C_1$-$C_4$-Alkylsulfonyl, $C_1$-$C_4$-Alkylsulfinyl oder $C_1$-$C_4$-Alkylthio

bedeuten.

Von besonderem Interesse sind auch erfindungsgemäße Verbindungen der Formel (I), worin

$R^3$ einen aliphatischen, araliphatischen oder aromatischen Rest der Formel

$$-CR^5R^6R^7$$

$R^5$, $R^6$ unabhängig voneinander Wasserstoff oder $C_1$-$C_4$-Alkyl oder $R^5$ und $R^6$ gemeinsam mit dem verbundenen C-Atom einen 5- oder 6-gliedrigen Ring und

$R^7$ Wasserstoff, Halogen, $C_1$-$C_4$-Alkyl, Phenyl oder Benzyl, vorzugsweise Wasserstoff oder Methyl

bedeuten.

Vorzugsweise ist $R^3$ Isopropyl, 2,2-Dimethyl-ethyl oder Cyclopentyl.

Von besonderem Interesse sind auch erfindungsgemäße Verbindungen der Formel (I), worin

$R^4$ Hydroxy, $-O^-Kat^+$, $C_1$-$C_{18}$-Alkoxy, $C_2$-$C_{18}$-Alkenyloxy, $C_2$-$C_{18}$-Alkinyloxy, wobei die letzgenannten drei Reste unsubstituiert oder durch ein oder mehrere Reste aus der Gruppe Halogen, $C_1$-$C_4$-Alkoxy, $C_1$-$C_4$-Alkylthio, ($C_1$-$C_4$-Alkoxy)-carbonyl, ($C_1$-$C_4$-Alkyl)-carbonyl, Phenyl, Phenoxy, Phenylthio, wobei die drei letztgenannten Reste unsubstituiert oder substituiert sind, Aminocarbonyl, ($C_1$-$C_4$-Alkyl)-aminocarbonyl und Di-($C_1$-$C_4$-alkyl)-aminocarbonyl substituiert sind, oder

Phenoxy, substituiertes Phenoxy, ($C_1$-$C_6$-Alkyliden)-aminoxy, $C_3$-$C_8$-Cycloalkyloxy oder Halogen oder

$R^4$ den Rest eines $\gamma$-Butyrolactons oder $\delta$-Valerianolactons, der die Bindungsstelle in $\alpha$-Position zur CO-Gruppe hat und unsubstituiert oder durch Halogen, $C_1$-$C_4$-Alkyl oder ($C_1$-$C_4$-Alkoxy)-carbonyl substituiert ist, oder

$R^4$ einen Rest der Formel

$$-D^1-CR^{12}R^{13}-D^2-L \text{ oder}$$

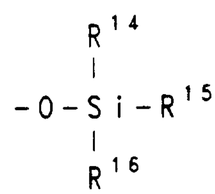

$Kat^+$ das Äquivalent eines Alkali- oder Erdalkalimetalls oder eines Ammoniumsalzes eines primären, sekundären, tertiären oder quartären Amins oder Di- oder Polyamins,

$R^{12}$, $R^{13}$ unabhängig voneinander Wasserstoff, $C_1$-$C_4$-Alkyl, $C_2$-$C_4$-Alkenyl, $C_2$-$C_4$-Alkinyl, Phenyl, substituiertes Phenyl, Benzyl, $C_1$-$C_4$-Alkoxy, $C_2$-$C_4$-Alkenyl, $C_2$-$C_4$-Alkinyloxy, Phe-

noxy oder substituiertes Phenoxy,

$R^{14}$, $R^{15}$, $R^{16}$ unabhängig voneinander $C_1$-$C_4$-Alkyl, $C_2$-$C_4$-Alkenyl oder $C_2$-$C_4$-Alkinyl, Phenyl oder substituiertes Phenyl,

$D^1$ O, S oder $NR^{17}$,

$R^{17}$ Wasserstoff oder $C_1$-$C_4$-Alkyl,

$D^2$ eine direkte Bindung oder eine Gruppe der Formel $CH_2$ oder $CH_2CH_2$,

L eine Säurederivat-Rest der Formel CO - O - $R^{18}$ oder CO - S - $R^{18}$, vorzugsweise CO - O - $R^{18}$ und

$R^{18}$ Wasserstoff, $C_1$-$C_{18}$-Alkyl, $C_2$-$C_8$-Alkenyl oder $C_2$-$C_8$-Alkinyl, wobei die letztgenannten 3 Reste unsubstituiert oder durch ein oder mehrere Reste aus der Gruppe Halogen substituiert sind, $C_3$-$C_8$-Cycloalkyl, $C_3$-$C_8$-Cycloalkenyl, Benzyl, Phenyl oder substituiertes Phenyl.

Vom besondere Interesse sind auch erfindungsgemäße Verbindungen der Formel (I), worin

$R^4$ Hydroxy, $O^-Kat^+$, $C_1$-$C_4$-Alkoxy, $C_2$-$C_4$-Alkenyloxy, $C_2$-$C_4$-Alkinyloxy, $C_3$-$C_6$-Cycloalkyloxy, Phenyloxy, ($C_1$-$C_4$-Alkyloxy)-$C_1$-$C_4$-alkoxy, ($C_1$-$C_4$-Alkyl)-carbonyl-$C_1$-$C_4$-alkoxy, $C_1$-$C_4$-Haloalkyloxy, $C_1$-$C_4$-Alkylidenaminoxy, ($C_1$-$C_4$-Alkylthio)-$C_1$-$C_4$-alkoxy, Phenyl-$C_1$-$C_4$-alkoxy, substituiertes Phenyl-$C_1$-$C_4$-alkoxy, Phenoxy-$C_1$-$C_4$-alkoxy, substituiertes Phenoxy-$C_1$-$C_4$-alkoxy, Phenylthio-$C_1$-$C_4$-alkoxy, substituiertes Phenylthio-$C_1$-$C_4$-alkoxy, wie Nitrophenylthio-$C_1$-$C_4$-alkoxy oder einen Benzyloxyrest, der durch ($C_1$-$C_4$)-Alkyl oder ($C_1$-$C_4$)-Alkoxy, Benzoxy oder Benzoxy, das durch $C_1$-$C_4$-Alkyl, $C_1$-$C_4$-Alkoxy, Halogen oder Nitro substituiert ist, substituiert ist, oder $R^4$ einen Rest der Formel

$D^1$-$CR^{12}R^{13}$-$D^2$-L ,

$Kat^+$ das Äquivalent eines Alkalimetalls, wie Natrium oder Kalium, oder eines Ammoniumsalzes eines primären, sekundären, tertiären oder quartären Amins,

$D^1$ O, S, NH, Methylamino oder Ethylamino,

$D^2$ eine direkte Bindung

$R^{12}$, $R^{13}$ unabhängig voneinander Wasserstoff, $C_1$-$C_4$-Alkyl, $C_2$-$C_4$-Alkenyl, $C_2$-$C_4$-Alkinyl, Phenyl oder Benzyl, vorzugsweise Wasserstoff oder Methyl,

L ein Säurederivat-Rest der Formel

- CO - O - $R^{18}$ oder - CO - S - $R^{18}$

$R^{18}$ Wasserstoff, $C_1$-$C_4$-Alkyl, $C_2$-$C_8$-Alkenyl, oder $C_2$-$C_4$-Alkinyl, Cyclohexyl, Cyclopentyl, Cyclohexenyl, Cyclopentenyl, Benzyl, Phenyl oder substituiertes Phenyl

bedeuten.

Bevorzugt sind erfindungsgemäße Verbindungen der Formel (I), worin

$R^4$ Hydroxy, $O^-Kat^+$ oder $C_1$-$C_4$-Alkoxy bedeutet.

Bevorzugt sind auch erfindungegemäße Verbindungen der Formel (I), worin die einzelnen Reste oder Gruppen zwei oder mehrere als bevorzugt bezeichnete Reste oder Gruppen enthalten.

Ein weiterer Gegenstand der vorliegenden Erfindung ist das Verfahren zur Herstellung von Verbindungen der allgemeinen Formel I, dadurch gekennzeichnet, daß man

(a) eine Verbindung der Formel (II)

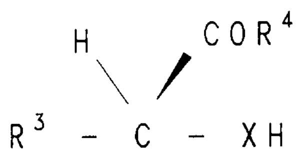

**(II),**

worin $R^3$, $R^4$ und X die bei Formel (I) genannten Bedeutungen haben, mit einer Verbindung der Formel (III)

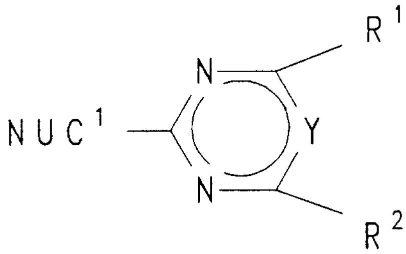

(III),

worin $NUC^1$ eine Abgangsgruppe bedeutet, beispielsweise aus der Gruppe Halogen, Alkylsulfonyl, Benzylsulfonyl und substituiertes Benzylsulfonyl, und $R^1$, $R^2$ und Y die bei Formel (I) genannte Bedeutung haben, in Gegenwart einer organischen oder anorganischen Base umsetzt,
(b) eine Verbindung der Formel (IV)

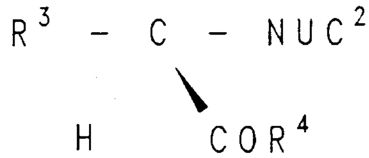

(IV)

worin $NUC^2$ eine Abgangsgruppe, beispielsweise aus der Gruppe Halogen, Tolylsulfonyloxy und Methylsulfonyloxy, bedeutet und $R^3$ und $R^4$ wie bei Formel (I) definiert sind,
mit einer Verbindung der Formel (V)

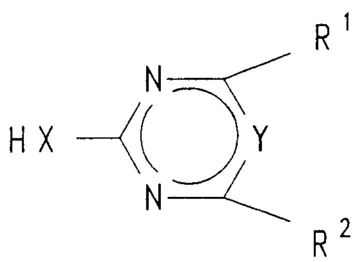

(V)

worin $R^1$, $R^2$, X und Y die bei Formel (I) genannten Bedeutungen haben, in Gegenwart einer anorganischen oder organischen Base umsetzt oder
c) für den Fall $R^4$ = OH oder $-O^-Kat^+$ in Formel (I), eine Verbindung der Formel (I), in der $R^4$ nicht Hydroxy oder $-O^-Kat^+$ ist, jedoch vorzugsweise ein Alkoxyrest ist,
mit Hilfe von Basen in Wasser oder Wasser/Alkoholgemischen zur freien Säure der Formel (I), worin $R^4$ Hydroxy bedeutet, umsetzt oder
d) für den Fall $R^4$ ungleich OH oder $-O^-Kat^+$ in Formel (I), eine Verbindung der Formel (I), in der $R^4$ Hydroxy bedeutet, mit Thionylchlorid, Oxalylchlorid, Chlorcarbonat, Carbonyldiimidazol oder Dicyclohexylcarbodiimid/4-(N,N-Dimethylamino)-pyridin in an sich bekannter Weise zu einem aktivierten Carbonsäurederivat umsetzt und letzteres mit einer Verbindung der Formel (VI),

$R^4$-H (VI)

worin
$R^4$ wie in Formel (I) definiert ist, ausgenommen die Bedeutungen Hydroxy und $-O^-Kat^+$, in Gegenwart einer anorganischen oder organischen Base zu Verbindungen der Formel (I) umsetzt, in der $R^4$ wie in Formel (VI) definiert ist, oder
e) ein Gemisch der L-Form der Formel (I) mit der entsprechenden D-Form, insbesondere ein racemisches Gemisch, einsetzt und danach ein Racemattrennverfahren durchführt oder
f) für den Fall $R^4$ ungleich OH oder $-O^-Kat^+$ in der Formel (I), eine Verbindung der Formel (I) in der $R^4$ Hydroxy bedeutet, mit einer Halogenverbindung der Formel (VII),

$R^0$-Hal (VII)

worin $R^0$ so definiert ist, daß $R^0$-O- die Bedeutung von $R^4$ nach Formel (I) hat, in Gegenwart einer anorganischen oder organischen Base umsetzt.

Als Basen für die Umsetzungen (a), (b), (d) und (f) sind beispielsweise anorganische Basen aus der Gruppe der Alkalicarbonate, wie $Na_2CO_3$, $K_2CO_3$, $NaHCO_3$ oder $KHCO_3$, Alkalihydride, wie NaH und KH, und Alkalifluoride, wie KF, oder organische Basen, beispielsweise tertiäre Amine wie Triethylamin oder DBU (1,8-Diazabicyclo[5.4.0]undec-7-en), geeignet.

Als Basen für die Umsetzung nach (c) sind beispielsweise anorganische, wasserlösliche Basen, wie Alkali- und Erdalkalimetallhydroxide, vorzugsweise Alkalimetallhydroxide wie NaOH oder KOH, geeignet.

In der Regel ist es vorteilhaft oder zweckmäßig, diese Umsetzungen in Gegenwart eines nichtwäßrigen, aprotischen Lösungsmittels durchzuführen. Als Lösungsmittel können bei diesen Umsetzungen Kohlenwasserstoffe, wie n-Heptan, Benzol, Toluol oder Xylol, halogenierte Kohlenwasserstoffe, wie $CH_2Cl_2$, $CHCl_3$ und $CCl_4$, Ether, wie Diethylether, Diglyme, Monoglyme, Dioxan und Tetrahydrofuran (THF), aprotisch polare Lösemittel, wie Dimethylformamid (DMF), N,N-Dimethylacetamid, Dimethylsulfoxid (DMSO) und Acetonitril, eingesetzt werden.

Auch kann es von Vorteil sein, die Umsetzungen in Gegenwart von Katalysatoren, beispielsweise von Kronenethern oder N,N,N',N'-Tetramethylendiamin, durchzuführen.

Die Ausgangssubstanzen der Formel II-VI sind bekannt oder lassen sich analog bekannten Verfahren synthetisieren; vgl. EP-A-347811, EP-A-400369, EP-A-409368, EP-A-400741 und dort zitierte Druckschriften; insbesondere sind optisch aktive Verbindungen der Formel (II) nach den Verfahren herzustellen, die J. Org. Chem. 1991 56, 2499; Tetrahedron 35, 1601-1605(1979), Tetrahedron 32, 1101-1106 (1976), Tetrahedron Lett. 1975, 2187-2190, Bull. Chem. Soc. Jpn. 1968, 41, 2178 beschrieben oder zitiert sind; optisch aktive Verbindungen der Formel (IV) können unter anderem aus den optisch aktiven Verbindungen der Formel (II), worin X ein Sauerstoffatom ist, unter üblichen Bedingungen durch Umwandlung der 2-Hydroxygruppe in eine Abgangsgruppe hergestellt werden, beispielsweise mittels Austausch- oder Veresterungsreaktionen. Die optisch aktiven Verbindungen der Formeln (II) und (IV) sind auch durch Racematspaltung aus den optisch inaktiven Gemischen der Enantiomeren zugänglich.

Die optisch aktiven Verbindungen der Formeln (I) sind ebenfalls durch Racematspaltung aus den optisch inaktiven Gemischen der Enantiomeren zugänglich. Als Racemattrennmethode ist beispielsweise auf der Stufe der racemischen Verbindungen, in denen $R^4$ = Hydroxy bedeutet, durch Salzbildung mit optisch aktiven Aminen, wie Brucin und Strychnin, und anschließender Trennung der gebildeten diastereomeren Salze möglich. Alternativ sind Trennungen der Enantiomere durch Chromatographie über spezielle Trennmaterialien, die mit optisch aktiven Verbindungen modifiziert worden sind, möglich. Zu Verfahren der Racemattrennung siehe z. B. in Houben-Weyl "Methoden der organischen Chemie" Bd. 11/2, S. 316 ff.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem

zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- une Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluent and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon`s "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Feffalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsuffonat oder nichtionische Emulgatoren wie Fettsäurepolyglykolester, Alkylarylpolyglykolether, Feftalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdisbergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemaßen Wirkstoffe in Mischungsformulierungen oder im Tank-Mix sind beispielsweise bekannte Wirkstoffe einsetrbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):

acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmedipham;desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsuffuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitrol; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl)-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyflourfen, paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d. h. 2-[[7-[2-Chlor-4-(trifluor-methyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

A. Chemische Beispiele

I) L-2-(4,6-Dimethoxypyrimidin-2-yloxy)-3-methylbutansäuremethylester (Bsp. 2 der Tabelle 1)

Man rührt eine Mischung von 4,4 g (0,015 Mol) 2-Benzylsulfonyl-4,6-dimethoxypyrimidin, 2,0 g (0,015 Mol) L-$\alpha$-Hydroxyisovaleriansäure und 2,10 g (0,015 Mol) Kaliumcarbonat in 40 ml Dimethylsulfoxid 4 h bei 100° C. Zur Aufarbeitung gibt man die Reaktionslösung auf Eiswasser und erhält nach Absaugen 2,5 g (61% d. Th.) L-2-(4,6-Dimethoxypyrimidin-2-yloxy)-3-methylbutansäuremethylester in Form von farblosen Kristallen vom Schmp. 30 - 32 °C. Der Drehwert des polarisierten Lichtes beträgt $[\alpha]_D^{20}$ = -31,49°.

II) L-2-(4,6-Dimethoxypyrimidin-2-yloxy)-3-methylbutansäure (Beispiel 1 der Tabelle 1)

1,3 g (0,005 Mol) L-2-(4,6-Dimethoxypyrimidin-2-yloxy)-3-methylbutansäuremethylester werden mit 10 ml konzentrierter Natronlauge in einem Gemisch von 50 ml Wasser und 30 ml Methanol auf dem Dampfbad erwärmt und anschließend 1 h gerührt. Nach Zugabe von Wasser und Ansäuern saugt man ab und erhält 0,7 g (58% d. Th.) L-2-(4,6-Dimethorypyrimidin-2-yloxy)-3-methylbutansäure in Form von farblosen Kristallen vom Schmp. 126 - 128 °C. Der Drehwert der Verbindung beträgt $[\alpha]_D^{20}$ = -18,23 °.

Analog den Verfahrensbeispielen I) und II) oder den Verfahren, wie sie in weiter oben beschrieben sind werden die anderen Verbindungen aus Tabelle 1 erhalten.

In der Tabelle 1 bedeutet Ph = Phenyl.

Tabelle 1: Verbindungen der Formel (Ia)

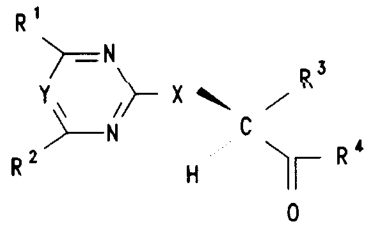

| Bsp. Nr. | $R^1$ | $R^2$ | $R^3$ | $R^4$ | X | Y | Schmp. (°C) bzw. [$n_D^{20}$] |
|---|---|---|---|---|---|---|---|
| 1 | $OCH_3$ | $OCH_3$ | i-Propyl | OH | O | CH | 126-128 $[\alpha]_D^{20}$ = -18,23° |
| 2 | " | " | " | $OCH_3$ | " | " | 30-32 $[\alpha]_D^{20}$ = -31,49° |
| 3 | " | " | " | $OC_2H_5$ | " | " | 45-47 $[\alpha]_D^{20}$ = -33,95° |
| 4 | " | " | " | $OC_3H_7$ (n) | " | " | |
| 5 | " | " | " | $OC_3H_7$ (i) | " | " | |
| 6 | " | " | " | $OC_4H_9$ (n) | " | " | |
| 7 | " | " | " | $OCH_2SCH_3$ | " | " | 1,5109 $[\alpha]_D^{20}$ = -82,67° |
| 8 | " | " | " | $OCH_2CO_2CH_3$ | " | " | |
| 9 | " | " | " | $OCH_2Ph$ | " | " | |
| 10 | " | " | " | $O-N=C(CH_3)_2$ | " | " | 1,4890 $[\alpha]_D^{20}$ = -45,95° |
| 11 | " | " | " | $OCH_2CH=CH_2$ | " | " | 36-38 $[\alpha]_D^{20}$ = -30,36° |
| 12 | " | " | " | $OCH_2C\equiv CH$ | " | " | 39-41 $[\alpha]_D^{20}$ = -45,04° |
| 13 | " | " | " | OH | " | N | |
| 14 | " | " | " | $OCH_3$ | " | " | |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 15 | " | " | " | $OC_2H_5$ | " | " | |
| 16 | " | " | " | $OC_3H_7$ (n) | " | " | |
| 17 | " | " | " | $OC_3H_7$ (i) | " | " | |
| 18 | " | " | " | $OC_4H_9$ (n) | " | " | |
| 19 | " | " | " | $OCH_2SCH_3$ | " | " | |
| 20 | " | " | " | $OCH_2CO_2CH_3$ | " | " | |
| 21 | " | " | " | $OCH_2Ph$ | " | " | |
| 22 | " | " | " | $O\text{-}N=C(CH_3)_2$ | " | " | |
| 23 | " | " | " | $OCH_2CH=CH_2$ | " | " | |
| 24 | " | " | " | $OCH_2C\equiv CH$ | " | " | |
| 25 | $OCH_3$ | $OCH_3$ | Cyclo-pentyl | OH | " | CH | |
| 26 | " | " | " | $OCH_3$ | " | " | |
| 27 | " | " | " | $OC_2H_5$ | " | " | |
| 28 | " | " | " | $OC_3H_7$ (n) | " | " | |
| 29 | " | " | " | $OC_3H_7$ (i) | " | " | |
| 30 | " | " | " | $OC_4H_9$ (n) | " | " | |
| 31 | " | " | " | $OCH_2SCH_3$ | " | " | |
| 32 | " | " | " | $OCH_2CO_2CH_3$ | " | " | |
| 33 | " | " | " | $OCH_2Ph$ | " | " | |
| 34 | " | " | " | $O\text{-}N=C(CH_3)_2$ | " | " | |
| 35 | " | " | " | $O\text{-}CH_2CH=CH_2$ | " | " | |
| 36 | " | " | " | $OCH_2C\equiv CH$ | " | " | |
| 37 | " | " | " | OH | O | N | |
| 38 | " | " | " | $OCH_3$ | " | " | |
| 39 | " | " | " | $OC_2H_5$ | " | " | |
| 40 | " | " | " | $OC_3H_7$ (n) | " | " | |
| 41 | " | " | " | $OC_3H_7$ (i) | " | " | |
| 42 | " | " | " | $OC_4H_9$ (n) | " | " | |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 43 | " | " | " | $OCH_2SCH_3$ | " | " | |
| 44 | " | " | " | $OCH_2CO_2CH_3$ | " | " | |
| 45 | " | " | " | $OCH_2Ph$ | " | " | |
| 46 | " | " | " | $O\text{-}N=C(CH_3)_2$ | " | " | |
| 47 | " | " | " | $OCH_2CH=CH_2$ | " | " | |
| 48 | " | " | " | $OCH_2C\equiv CH$ | " | " | |
| 49 | " | " | t-Butyl | OH | O | CH | 145-147 $[\alpha]_D^{20}$ = -17,99° |
| 50 | " | " | " | $OCH_3$ | " | " | 120-122 $[\alpha]_D^{20}$ = -33,76° |
| 51 | " | " | " | $OC_2H_5$ | " | " | 102-104 $[\alpha]_D^{20}$ = -33,50° |
| 52 | " | " | " | $OC_3H_7$ (n) | " | " | |
| 53 | " | " | " | $OC_3H_7$ (i) | " | " | |
| 54 | " | " | " | $OC_4H_9$ (n) | " | " | |
| 55 | " | " | " | $OCH_2SCH_3$ | " | " | 92-94 $[\alpha]_D^{20}$ = -66,59° |
| 56 | " | " | " | $OCH_2CO_2CH_3$ | " | " | |
| 57 | " | " | " | $OCH_2Ph$ | " | " | |
| 58 | " | " | " | $O\text{-}N=C(CH_3)_2$ | " | " | |
| 59 | " | " | " | $OCH_2CH=CH_2$ | " | " | |
| 60 | " | " | " | $OCH_2C\equiv CH$ | " | " | |
| 61 | " | " | " | OH | O | N | |
| 62 | " | " | " | $OCH_3$ | " | " | |
| 63 | " | " | " | $OC_2H_5$ | " | " | |
| 64 | " | " | " | $OC_3H_7$ (n) | " | " | |
| 65 | " | " | " | $OC_3H_7$ (i) | " | " | |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 66 | " | " | " | $OC_4H_9$ (n) | " | " | |
| 67 | " | " | " | $OCH_2SCH_3$ | " | " | |
| 68 | " | " | " | $OCH_2CO_2CH_3$ | " | " | |
| 69 | " | " | " | $OCH_2Ph$ | " | " | |
| 70 | " | " | " | $O\text{-}N=C(CH_3)_2$ | " | " | |
| 71 | " | " | " | $OCH_2CH=CH_2$ | " | " | |
| 72 | " | " | " | $OCH_2C\equiv CH$ | " | " | |
| 73 | " | " | Cyclo-hexyl | OH | O | CH | 171-173 $[\alpha]_D^{20}$ = -16,58° |
| 74 | " | " | " | $OCH_3$ | " | " | 98-100 $[\alpha]_D^{20}$ = -30,33° |
| 75 | " | " | " | $OC_2H_5$ | " | " | |
| 76 | " | " | " | $OC_3H_7$ (n) | " | " | |
| 77 | " | " | " | $OC_3H_7$ (i) | " | " | |
| 78 | " | " | " | $OC_4H_9$ (n) | " | " | |
| 79 | " | " | " | $OCH_2SCH_3$ | " | " | |
| 80 | " | " | " | $OCH_2CO_2CH_3$ | " | " | |
| 81 | " | " | " | $OCH_2Ph$ | " | " | |
| 82 | " | " | " | $O\text{-}N=C(CH_3)_2$ | " | " | |
| 83 | " | " | " | $OCH_2CH=CH_2$ | " | " | |
| 84 | " | " | " | $OCH_2C\equiv CH$ | " | " | |
| 85 | " | " | " | OH | O | N | |
| 86 | " | " | " | $OCH_3$ | " | " | |
| 87 | " | " | " | $OC_2H_5$ | " | " | |
| 88 | " | " | " | $OC_3H_7$ (n) | " | " | |
| 89 | " | " | " | $OC_3H_7$ (i) | " | " | |
| 90 | " | " | " | $OC_4H_9$ (n) | " | " | |
| 91 | " | " | " | $OCH_2SCH_3$ | " | " | |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 92 | $OCH_3$ | $OCH_3$ | Cyclo-hexyl | $OCH_2CO_2CH_3$ | O | N | |
| 93 | " | " | " | $OCH_2Ph$ | " | " | |
| 94 | " | " | " | $O\text{-}N{=}C(CH_3)_2$ | " | " | |
| 95 | " | " | " | $OCH_2CH{=}CH_2$ | " | " | |
| 96 | " | " | " | $OCH_2C{\equiv}CH$ | " | " | |
| 97 | " | " | sec-Butyl | OH | O | CH | 114-116 $[\alpha]_D^{20}$ = -13,2° |
| 98 | " | " | " | $OCH_3$ | " | " | Oel $[\alpha]_D^{20}$ = -30,9° |
| 99 | " | " | " | $OC_2H_5$ | " | " | Oel $[\alpha]_D^{20}$ = -30,4° |
| 100 | " | " | " | $OC_3H_7$ (n) | " | " | |
| 101 | " | " | " | $OC_3H_7$ (i) | " | " | |
| 102 | " | " | " | $OC_4H_9$ (n) | " | " | |
| 103 | " | " | " | $OCH_2SCH_3$ | " | " | |
| 104 | " | " | " | $OCH_2CO_2CH_3$ | " | " | |
| 105 | " | " | " | $OCH_2Ph$ | " | " | |
| 106 | " | " | " | $O\text{-}N{=}C(CH_3)_2$ | " | " | |
| 107 | " | " | " | $OCH_2CH{=}CH_2$ | " | " | |
| 108 | " | " | " | $OCH_2C{\equiv}CH$ | " | " | |
| 109 | " | " | " | OH | O | N | |
| 110 | " | " | " | $OCH_3$ | " | " | |
| 111 | " | " | " | $OC_2H_5$ | " | " | |
| 112 | " | " | " | $OC_3H_7$ (n) | " | " | |
| 113 | " | " | " | $OC_3H_7$ (i) | " | " | |
| 114 | " | " | " | $OnC_4H_9$ (n) | " | " | |
| 115 | " | " | " | $OCH_2SCH_3$ | " | " | |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 116 | " | " | " | $OCH_2CO_2CH_3$ | " | " | |
| 117 | " | " | " | $OCH_2Ph$ | " | " | |
| 118 | " | " | " | $O\text{-}N{=}C(CH_3)_2$ | " | " | |
| 119 | " | " | " | $OCH_2CH{=}CH_2$ | " | " | |
| 120 | " | " | " | $OCH_2C{\equiv}CH$ | " | " | |
| 121 | " | " | " | $O^-K^+$ | " | CH | 200 $[\alpha]_D^{20}$ = -0,15° |
| 122 | " | " | " | $O^-Li^+$ | " | " | $[\alpha]_D^{20}$ = +0,42° |
| 123 | " | " | " | $O^-[HN(C_2H_5)_3]^+$ | " | " | $[\alpha]_D^{20}$ = -3,8° |
| 124 | " | " | iso-$C_3H_7$ | $O^-Na^+$ | " | " | 242-244 $[\alpha]_D^{20}$ = -1,22° |
| 125 | " | " | " | $O^-K^+$ | " | " | 220-222 $[\alpha]_D^{20}$ = -0,76° |
| 126 | " | " | " | $O^-Li^+$ | " | " | 224-226 $[\alpha]_D^{20}$ = -1,43° |
| 127 | " | " | " | $O^-NH_4^+$ | " | " | |
| 128 | $OCH_3$ | $OCH_3$ | sec-$C_4H_9$ | $O^-Na^+$ | O | CH | 200-202 $[\alpha]_D^{20}$ = -0,2° |
| 129 | " | " | iso-$C_3H_7$ | $O^-[HN(C_2H_5)_3]^+$ | " | " | 1,4858 $[\alpha]_D^{20}$ = -5,37° |
| 130 | " | " | tert-$C_4H_9$ | $O^-Li^+$ | " | " | 175-177 $[\alpha]_D^{20}$ = +1,83° |
| 131 | " | " | " | $O^-Na^+$ | " | " | 226-228 $[\alpha]_D^{20}$ = +1,34° |

| Bsp. Nr. | $R^1$ | $R^2$ | $R^3$ | $R^4$ | X | Y | Schmp. (°C) bzw. $[n_D^{20}]$ |
|---|---|---|---|---|---|---|---|
| 132 | " | " | iso-$C_3H_7$ | $O-CH_2CO_2C_2H_5$ | " | " | 70-72 $[\alpha]_D^{20}$ = -43,11° |

B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetrbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277° C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

| | |
|---|---|
| 75 Gewichtsteile | einer Verbindung der Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | |
|---|---|
| 25 Gewichtsteil(e) | einer Verbindung der Formel (I), |
| 5 " | 2,2'dinaphthylmethan-6,6'-disulfonsaures Natrium |
| 2 " | oleoylmethyltaurinsaures Natrium |
| 1 " | Polyvinylalkohol, |
| 17 " | Calciumcarbonat und |
| 50 " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen (L-Formen) sowie die entsprechenden D-Formen und Racemate werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 1, 2, 3, 7, 10, 11, 12, 49, 50, 51, 55, 97, 98, 99, 121, 122, 123, 124, 125, 126, 128, 129, 130, 131 und 132 aus Tabelle 1 sehr gute herbizide Wirkung gegen Schadpflanzen wie Alopecurus myosuroides, Amaranthus retroflexus, Avena sativa, Poa annua, Setaria pumila, Sinapis alba, Stellaria media, Echinochloa crus-galli und Lolium multiflorum im Vorauflaufverfahren bei einer Aufwandmenge von 0,3 kg bis 0,005 kg Aktivsubstanz pro Hektar. Bei gleicher Aufwandmenge zeigen dagegen die Versuche mit der D-Form als auch mit den racemischen Mischungen der D- und L-Form der genannten Verbindungen wesentlich geringere Vorauflaufwirkungen bei den genannten Testpflanzen.

2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen (L-Formen) und in Vergleichsversuchen die entsprechenden D-Formen und Racemate werden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 1, 2, 3, 7, 10, 11, 12, 49, 50, 51, 55, 97, 98, 99, 121, 122, 123, 124, 125, 126, 128, 129, 130, 131 und 132 aus Tabelle 1 sehr gute herbizide Wirkung gegen Schadplanzen wie Alopecurus myosuroides, Amaranthus retroflexus, Avena sativa, Poa annua, Setaria pumila, Sinapis alba, Stellaria media, Echinochloa crus-galli und Lolium multiflorum im Nachauflautuerfahren bei einer Aufwandmenge von 0,3 kg bis 0,005 kg Aktivsubstanz pro Hektar. Bei gleicher Aufwandmenge zeigen dagegen die Versuche mit der D-Form als auch mit den racemischen Mischungen der D- und L-Form der genannten Verbindungen wesentlich geringere Nachlaufwirkungen bei den genannten Testpflanzen.

**Patentansprüche**

**1.** Verbindungen der Formel (I),

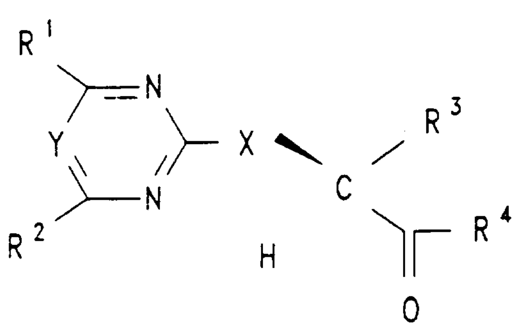

(I)

worin

| | |
|---|---|
| X | O oder S, |
| Y | N oder CH, |
| $R^1$, $R^2$ | unabhängig voneinander Wasserstoff, Alkyl, Alkoxy, Alkylthio, Halogen, Haloalkoxy, Amino, Alkylamino oder Dialkylamino, |

$R^3$ ein aliphatischer oder araliphatischer Rest der Formel

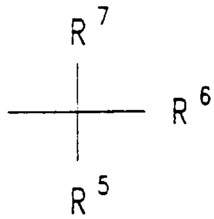

oder

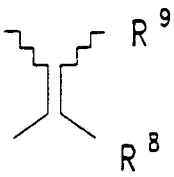

$R^5$, $R^6$ unabhängig voneinander Wasserstoff oder Alkyl oder $R^5$ und $R^6$ gemeinsam mit dem verbundenen C-Atom einen 3- bis 6-gliedrigen Ring, der ein Sauerstoffatom enthalten kann und durch ein oder mehrere Alkylgruppen substituiert sein kann,

$R^7$ Wasserstoff, Halogen, Alkyl, das unsubstituiert oder durch ein oder mehrere Reste aus der Gruppe Halogen, Hydroxy, Alkoxy und Alkylthio substituiert ist, oder Cycloalkyl Hydroxy, Cyano, Aryl oder Arylalkyl, wobei die letztgenannten beiden Reste im Aryl-Teil unsubstituiert oder substituiert sind,

$R^8$ Alkyl, Phenyl oder substituiertes Phenyl oder $R^8$ gemeinsam mit $R^{10}$ und der verbundenen Ethenylen-Gruppe einen Cyclopenten- oder Cyclohexenring, der unsubstituiert oder durch ein oder mehrere Alkylgruppen substituiert ist,

$R^9$ Wasserstoff oder Alkyl,

$R^{10}$ unabhängig voneinander Wasserstoff oder Alkyl oder $R^{10}$ gemeinsam mit $R^8$ und der verbundenen Ethenylen-Gruppe einen Cyclopenten- oder Cyclohexenring, der unsubstituiert oder durch ein oder mehrere Alkylgruppen substituiert ist,

$R^{11}$ Wasserstoff, Halogen, Nitro, Alkyl, Haloalkyl, Alkoxy, Alkylsulfonyl, Alkylsulfinyl oder Alkylthio,

$R^4$ Hydroxy, $-O^-Kat^+$, einen aliphatischen gesättigten oder ein- oder mehrfach ungesättigten Kohlenwasserstoffoxyrest
wobei der Rest unsubstituiert oder durch ein oder mehrere Reste aus der Gruppe Halogen, Alkoxy, Alkylthio, Alkoxycarbonyl, Alkylcarbonyl, Phenyl, Phenoxy, Phenylthio, wobei die drei letztgenannten Reste unsubstituiert oder substituiert sind, Aminocarbonyl,
Alkylaminocarbonyl und Dialkylaminocarbonyl substituiert ist, oder Phenoxy, substituiertes Phenoxy, Alkylidenaminoxy, Cycloalkyloxy oder Halogen oder
$R^4$ den Rest eines 5- oder 6-Ringlactons, oder
$R^4$ einen Rest der Formel

$-D^1-CR^{12}R^{13}-D^2-L$ oder

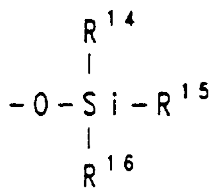

$Kat^+$ das Äquivalent eines ein-, zwei- oder mehrwertigen Kations,

$R^{12}$, $R^{13}$ unabhängig voneinander Wasserstoff, Alkyl, Alkenyl, Alkinyl, Aryl, substituiertes Aryl, Benzyl, Alkoxy, Alkenyl, Alkinyloxy, Aryloxy oder substituiertes Aryloxy,

$R^{14}$, $R^{15}$, $R^{16}$ unabhängig voneinander Alkyl, Alkenyl oder Alkinyl, Phenyl oder substituiertes Phenyl,

$D^1$ O, S oder $NR^{17}$,

$R^{17}$ Wasserstoff oder Alkyl,

$D^2$ eine direkte Bindung oder eine gesättigte oder ungesättigte Kohlenwasserstoffgruppe,

L eine Säurederivat-Rest der Formel

- CO - O - $R^{18}$ oder - CO - S - $R^{18}$ und

$R^{18}$ Wasserstoff, Alkyl, Alkenyl oder Alkinyl, wobei die letztgenannten 3 Reste unsubstituiert oder durch ein oder mehrere Reste aus der Gruppe Halogen substituiert sind, Cycloalkyl, Cycloalkenyl, Benzyl, Phenyl oder substituiertes Phenyl

bedeuten, in einer Enantiomerenreinheit der L-Form von größer als Null.

**2.** Verbindungen gemäß Anspruch 1, dadurch gekennzeichnet, daß

$R^1$, $R^2$ unabhängig voneinander $C_1$-$C_4$-Alkyl, $C_1$-$C_4$-Alkoxy, Halogen, $C_1$-$C_4$-Haloalkoxy, $C_1$-$C_4$-Alkylamino oder Di-($C_1$-$C_4$-alkyl)-amino

bedeuten.

**3.** Verbindungen gemäß Anspruch 1 oder 2, dadurch gekennzeichnet, daß

$R^5$, $R^6$ unabhängig voneinander Wasserstoff oder $C_1$-$C_4$-Alkyl oder $R^5$ und $R^6$ gemeinsam mit dem verbundenen C-Atom einen 3- bis 6-gliedrigen Ring, der ein Sauerstoffatom enthalten kann und durch ein oder mehrere $C_1$-$C_4$-Alkyl substituiert sein kann,

$R^7$ Wasserstoff, Halogen, $C_1$-$C_4$-Alkyl, das unsubstituiert oder durch ein oder mehrere Reste aus der Gruppe Halogen, Hydroxy, $C_1$-$C_4$-Alkoxy und $C_1$-$C_4$-Alkylthio substituiert ist, oder $C_3$-$C_8$-Cycloalkyl, Hydroxy, Cyano, Thienyl, Naphthyl, Dihydronaphthyl, wobei die letztgenannten drei Reste unsubstituiert oder substituiert sind, oder einen Rest der Formel

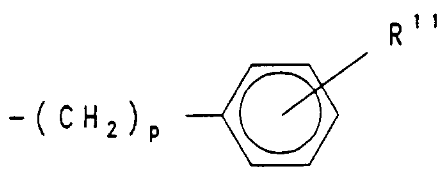

p 0, 1, 2 oder 3,

$R^8$ $C_1$-$C_4$-Alkyl, Phenyl oder substituiertes Phenyl oder $R^8$ gemeinsam mit $R^{10}$ und der verbundenen Ethenylen-Gruppe einen Cyclopenten- oder Cyclohexenring, der unsubstituiert oder durch ein oder mehrere $C_1$-$C_4$-Alkyl substituiert ist,

$R^9$ Wasserstoff oder $C_1$-$C_4$-Alkyl,

$R^{10}$ unabhängig voneinander Wasserstoff oder $C_1$-$C_4$-Alkyl oder $R^{10}$ gemeinsam mit $R^8$ und der verbundenen Ethenylen-Gruppe einen Cyclopenten- oder Cyclohexenring, der unsubstituiert oder durch ein oder mehrere $C_1$-$C_4$-Alkyl substituiert ist,

$R^{11}$ Wasserstoff, Halogen, Nitro, $C_1$-$C_{12}$-Alkyl, $C_1$-$C_4$-Haloalkyl, $C_1$-$C_4$-Alkoxy, $C_1$-$C_4$-Alkylsulfonyl, $C_1$-$C_4$-Alkylsulflnyl oder $C_1$-$C_4$-Alkylthio

bedeuten.

**4.** Verbindungen gemäß einem der Ansprüche 1 bis 3, dadurch gekennzeichnet, daß

$R^3$ einen aliphatischen, araliphatischen oder aromatischen Rest der Formel - $CR^5R^6R^7$

$R^5$, $R^6$ unabhängig voneinander Wasserstoff oder $C_1$-$C_4$-Alkyl oder $R^5$ und $R^6$ gemeinsam mit dem verbundenen C-Atom einen 5- oder 6-gliedrigen Ring und

$R^7$ Wasserstoff, Halogen, $C_1$-$C_4$-Alkyl, Phenyl oder Benzyl,

bedeuten.

**5.** Verbindungen gemäß einem der Ansprüche 1 bis 4, dadurch gekennzeichnet, daß

$R^4$ Hydroxy, $-O^-Kat^+$, $C_1$-$C_{18}$-Alkoxy, $C_2$-$C_{18}$-Alkenyloxy, $C_2$-$C_{18}$-Alkinyloxy, wobei die letzgenannten drei Reste unsubstituiert oder durch ein oder mehrere Reste aus der Gruppe Halogen, $C_1$-$C_4$-Alkoxy, $C_1$-$C_4$-Alkylthio, ($C_1$-$C_4$-Alkoxy)-carbonyl, ($C_1$-$C_4$-Alkyl)-carbonyl, Phenyl, Phenoxy, Phenylthio, wobei die drei letztgenannten Reste unsubstituiert oder substituiert sind, Aminocarbonyl, ($C_1$-$C_4$-Al-

kyl)aminocarbonyl und Di-($C_1$-$C_4$-alkyl)-aminocarbonyl substituiert sind, oder Phenoxy, substituiertes Phenoxy, ($C_1$-$C_6$-Alkyliden)-aminoxy, $C_3$-$C_8$-Cyoloalkyloxy oder Halogen oder
$R^4$ den Rest eines $\gamma$-Butyrolactons oder $\delta$-Valerianolactons, der die Bindungsstelle in $\alpha$-Position zur CO-Gruppe hat und unsubstituiert oder durch Halogen, $C_1$-$C_4$-Alkyl oder ($C_1$-$C_4$-Alkoxy)-carbonyl substituiert ist, oder
$R^4$ einen Rest der Formel

$-D^1-CR^{12}R^{13}-D^2-L$ oder

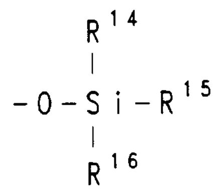

Kat$^+$ das Äquivalent eines Alkali- oder Erdalkalimetalls oder eines Ammoniumsalzes eines primären, sekundären, tertiären oder quartären Amins oder Di- oder Polyamins,
$R^{12}$, $R^{13}$ unabhängig voneinander Wasserstoff, $C_1$-$C_4$-Alkyl, $C_2$-$C_4$-Alkenyl, $C_2$-$C_4$-Alkinyl, Phenyl, substituiertes Phenyl, Benzyl, $C_1$-$C_4$-Alkoxy, $C_2$-$C_4$-Alkenyl, $C_2$-$C_4$-Alkinyloxy, Phenoxy oder substituiertes Phenoxy,
$R^{14}$, $R^{15}$, $R^{16}$ unabhängig voneinander $C_1$-$C_4$-Alkyl, $C_2$-$C_4$-Alkenyl oder $C_2$-$C_4$-Alkinyl, Phenyl oder substituiertes Phenyl,
$D^1$ O, S oder $NR^{17}$,
$R^{17}$ Wasserstoff oder $C_1$-$C_4$-Alkyl,
$D^2$ eine direkte Bindung oder eine Gruppe der Formel $CH_2$ oder $CH_2CH_2$,
L eine Säurederivat-Rest der Formel CO - O - $R^{18}$ oder CO - S - $R^{18}$, vorzugsweise CO - O -, $R^{18}$ und
$R^{18}$ Wasserstoff, $C_1$-$C_{18}$-Alkyl, $C_2$-$C_8$-Alkenyl oder $C_2$-$C_8$-Alkinyl, wobei die letztgenannten 3 Reste unsubstituiert oder durch ein oder mehrere Reste aus der Gruppe Halogen substituiert sind, $C_3$-$C_8$-Cycloalkyl, $C_3$-$C_8$-Cycloalkenyl, Benzyl, Phenyl oder substituiertes Phenyl

bedeuten.

**6.** Verbindungen gemäß einem der Ansprüche 1 bis 5, dadurch gekennzeichnet, daß
$R^4$ Hydroxy, $O^-Kat^+$, $C_1$-$C_4$-Alkoxy, $C_2$-$C_4$-Alkenyloxy, $C_2$-$C_4$-Alkinyloxy, $C_3$-$C_6$-Cycloalkyloxy, Phenyloxy, ($C_1$-$C_4$-Alkylxy)-$C_1$-$C_4$-alkoxy, ($C_1$-$C_4$-Alkyl)-carbonyl-$C_1$-$C_4$-alkoxy, $C_1$-$C_4$-Haloalkyloxy, $C_1$-$C_4$-Alkylidenaminoxy, ($C_1$-$C_4$-Alkylthio)-$C_1$-$C_4$-alkoxy, Phenyl-$C_1$-$C_4$-alkoxy, substituiertes Phenyl-$C_1$-$C_4$-alkoxy, Phenoxy-$C_1$-$C_4$-alkoxy, substituiertes Phenoxy-$C_1$-$C_4$-alkoxy, Phenylthio-$C_1$-$C_4$-alkoxy, substituiertes Phenylthio-$C_1$-$C_4$-alkoxy oder einen Benzyloxyrest, der durch ($C_1$-$C_4$)-Alkyl oder ($C_1$-$C_4$)-Alkoxy, Benzoxy oder Benzoxy, das durch $C_1$-$C_4$-Alkyl, $C_1$-$C_4$-Alkoxy, Halogen oder Nitro substituiert ist, substituiert ist,oder
$R^4$ einen Rest der Formel

$D^1-CR^{12}R^{13}-D^2-L$ ,

Kat$^+$ das Äquivalent eines Alkalimetalls oder eines Ammoniumsalzes eines primären, sekundären, tertiären oder quartären Amins,
$D^1$ O, S, NH, Methylamino oder Ethylamino,
$D^2$ eine direkte Bindung
$R^{12}$, $R^{13}$ unabhängig voneinander Wasserstoff, $C_1$-$C_4$-Alkyl, $C_2$-$C_4$-Alkenyl, $C_2$-$C_4$-Alkinyl, Phenyl oder Benzyl, vorzugsweise Wasserstoff oder Methyl,

L ein Säurederivat-Rest der Formel

- CO - O - $R^{18}$ oder - CO - S - $R^{18}$

$R^{18}$ Wasserstoff, $C_1$-$C_4$-Alkyl, $C_2$-$C_8$-Alkenyl, oder $C_2$-$C_4$-Alkinyl, Cyclohexyl, Cyclopentyl, Cyclohexenyl, Cyclopentenyl, Benzyl, Phenyl oder substituiertes Phenyl

bedeuten.

**7.** Verfahren zur Herstellung von Verbindungen der Formel (I) gemäß Anspruch 1, dadurch gekennzeichnet, daß man

(a) eine Verbindung der Formel (II)

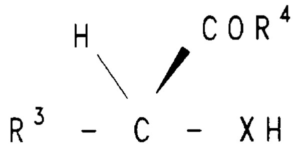

(II),

worin $R^3$, $R^4$ und X die bei Formel (I) genannte Bedeutungen haben, mit einer Verbindung der Formel (III)

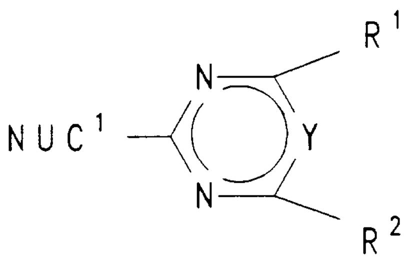

(III),

worin $NUC^1$ eine Abgangsgruppe bedeutet und $R^1$, $R^2$ und Y die bei Formel (I) genannte Bedeutung haben, in Gegenwart einer organischen oder anorganischen Base umsetzt,

(b) eine Verbindung der Formel (IV)

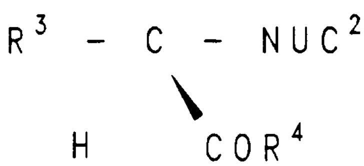

(IV)

worin $NUC^2$ eine Abgangsgruppe bedeutet und $R^3$ und $R^4$ wie bei Formel (I) definiert sind, mit einer Verbindung der Formel (V)

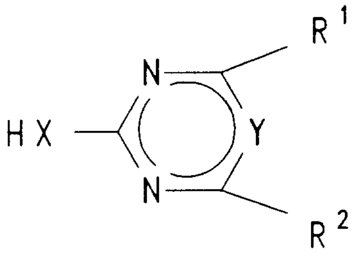

(V),

worin $R^1$, $R^2$, X und Y die bei Formel (I) genannten Bedeutungen haben, in Gegenwart einer

anorganischen oder organischen Base umsetzt oder
c) für den Fall $R^4$ = OH oder -$O^-Kat^+$ in Formel (I), eine Verbindung der Formel (I), in der $R^4$ nicht Hydroxy oder -$O^-Kat^+$ ist,
mit Hilfe von Basen in Wasser oder Wasser/Alkoholgemischen zur freien Säure der Formel (I), worin $R^4$ Hydroxy bedeutet, umsetzt oder
d) für den Fall $R^4$ ungleich OH oder -$O^-Kat^+$ in Formel (I), eine Verbindung der Formel (I), in der $R^4$ Hydroxy bedeutet, mit Thionylchlorid, Oxalylchlorid, Chlorcarbonat, Carbonyldiimidazol oder Dicyclohexylcarbodiimid/4-(N,N-Dimethylamino)-pyridin in an sich bekannter Weise zu einem aktivierten Carbonsäurederivat umsetzt und letzteres mit einer Verbindung der Formel (VI)

$R^4$-H (VI),

worin
$R^4$ wie in Formel (I) definiert ist, ausgenommen die Bedeutungen Hydroxy und -$O^-Kat^+$, in Gegenwart einer anorganischen oder organischen Base zu Verbindungen der Formel (I) umsetzt, in der $R^4$ wie in Formel (VI) definiert ist, oder
e) ein Gemisch der L-Form der Formel (I) mit der entsprechenden D-Form, einsetzt und daran ein Racemattrennverfahren durchführt oder
f) für den Fall $R^4$ ungleich OH oder -$O^-Kat^+$ in der Formel (I), eine Verbindung der Formel (I), in der $R^4$ Hydroxy bedeutet, mit einer Halogenverbindung der Formel (VII),

$R^0$-Hal (VII)

worin $R^0$ so definiert ist, daß $R^0$-O- die Bedeutung von $R^4$ nach Formel (I) hat, in Gegenwart einer anorganischen oder organischen Base umsetzt.

**8.** Herbizide und pflanzenwachstumsregulierende Mittel, dadurch gekennzeichnet, daß sie eine Verbindung der Formel (I) nach einem der Ansprüche 1 bis 6 und übliche Formulierungshilfsmittel enthalten.

**9.** Verwendung der Verbindungen der Formel (I) nach einem der Ansprüche 1 bis 6 als Herbizide oder Pflanzenwachstumsregulatoren.

**10.** Verfahren zur Bekämpfung von Schadpflanzen oder zur Wachstumsregulierung von Pflanzen, dadurch gekennzeichnet, daß man eine oder mehrere Verbindungen der Formel (I) nach einem der Ansprüche 1 bis 6 auf die Pflanzen, Pflanzenteile, Pflanzensamen oder die Fläche, auf der die Pflanzen wachsen, appliziert.

Europäisches Patentamt

# EUROPÄISCHER RECHERCHENBERICHT

Nummer der Anmeldung

EP 93 10 4654

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 409 368 (SCHERING)<br>*EXAMPLES 58-61,63,65-67,72,75,96-101,103 *<br>* Ansprüche *<br>--- | 1-10 | C07D239/60<br>C07D251/30<br>C07D405/12<br>A01N43/54 |
| D,X | EP-A-0 400 741 (SHELL)<br>* Seite 9 - Seite 36; Ansprüche *<br>--- | 1-10 | |
| D,X | EP-A-0 347 811 (KUMIAI)<br>* Seite 5 - Seite 28; Ansprüche *<br>--- | 1-10 | |
| A | CHEMICAL ABSTRACTS, vol. 116, no. 28, 1992, Columbus, Ohio, US;<br>abstract no. 59390u,<br>Seite 865 ;Spalte 2 ;<br>* Zusammenfassung * | 1-10 | |
| A | & JP-A-03 200 772 (KUMIAI)<br>2. September 1991<br>--- | 1-10 | |
| A | CHEMICAL ABSTRACTS, vol. 117, no. 28, 1992, Columbus, Ohio, US;<br>abstract no. 48615r,<br>Seite 943 ;Spalte 1 ;<br>* Zusammenfassung * | 1-10 | **RECHERCHIERTE SACHGEBIETE (Int. Cl.5)** |
| A | & JP-A-03 240 777 (UBE)<br>28. Oktober 1991<br>--- | 1-10 | C07D |
| P,X | EP-A-0 517 215 (UBE)<br>9. Dezember 1992<br>* Seite 32 - Seite 42; Ansprüche *<br>--- | 1-10 | |
| P,X | EP-A-0 481 512 (UBE)<br>22. April 1992<br>* Seite 11 - Seite 33; Ansprüche *<br>----- | 1-10 | |

Der vorliegende Recherchenbericht wurde für alle Patentansprüche erstellt

| Recherchenort | Abschlußdatum der Recherche | Prüfer |
|---|---|---|
| DEN HAAG | 29 APRIL 1993 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)